**Europäisches Patentamt**

**European Patent Office**

**Office européen des brevets**

(11) Veröffentlichungsnummer : **0 105 943**
**B1**

# EUROPÄISCHE PATENTSCHRIFT

(45) Veröffentlichungstag der Patentschrift :
12.02.86

(21) Anmeldenummer : 82109327.5

(22) Anmeldetag : 08.10.82

(51) Int. Cl.⁴ : **C 12 M    1/00**

(54) Verfahren und Vorrichtung zur Gewinnung von Biogas aus einer Biogasmasse.

(43) Veröffentlichungstag der Anmeldung :
25.04.84 Patentblatt 84/17

(45) Bekanntmachung des Hinweises auf die Patenterteilung : 12.02.86 Patentblatt 86/07

(84) Benannte Vertragsstaaten :
AT BE CH DE FR GB IT LI NL SE

(56) Entgegenhaltungen :
FR-A-    874 378
FR-A- 2 480 062

(73) Patentinhaber : Ökotherm Vertriebs-GmbH
Hauptstrasse 1
D-2357 Hagen (DE)

(72) Erfinder : Jendrike, Helmut
Hauptstrasse 1
D-2357 Hagen (DE)

(74) Vertreter : Lorenz, Eduard et al
Rechtsanwälte Lorenz, Eduard - Seidler, Bernhard
Seidler, Margrit - Gossel, Hans-K. Philipps, Ina, Dr.
Widenmayerstrasse 23
D-8000 München 22 (DE)

Jouve, 18, rue St-Denis, 75001 Paris, France

**Beschreibung**

Die Erfindung betrifft ein Verfahren zur Gewinnung von Biogas aus einer Biomasse, wie Gülle, einem Kot, Schlachtereiabfällen oder dergleichen, und eine Flüssigkeit enthaltendem Gemisch, durch anaeroben Abbau in einem alkalischen Medium in einem geschlossenen Raum, bei dem die sich unten ablagernde schwere Biomassesinkschicht abgesaugt und auf die sich oben bildende Biomasseschwimmschicht aufgebracht wird, sowie eine Vorrichtung zur Durchführung des Verfahrens.

Der Kreislauf des Kohlenstoffs in der Natur ist im wesentlichen gekennzeichnet durch Fixierung des in der Luft enthaltenen Kohlenstoffdioxids in Form von organischen Verbindungen, die in der Pflanze bei der Fotosynthese gebildet werden. Der größte Teil der erzeugten Biomasse zerfällt in der Rückreaktion an der Luft meist mit Hilfe aerober Bakterien wieder in Kohlenstoffdioxid und Wasser. Unter anaeroben Bedingungen und im neutralen bis schwach alkalischen pH-Bereich ist es jedoch möglich, den Abbau so zu steuern, daß das entstehende Gas überwiegend zu zwei Dritteln aus Methan und zu einem Drittel aus $CO_2$ besteht. Daneben werden noch geringe Mengen von $H_2S$ und $NH_3$ gebildet. Der Rückstand findet dann Verwendung als hochwertiger Dünger.

Während der Verarbeitung der Gülle (im wesentlichen ein Gemisch aus Kot, Harn, Kurzstroh und Wasser) zu Biogas ändert sich je nach fortschreitendem Verarbeitungsverlauf das spezifische Gewicht der Biomasse. Es werden bei der Gasbildung einerseits Feststoffteile der Biomasse durch stark gasende Bläschen in den oberen Teil eines Biogas- bzw. Faulbehälters transportiert. Diese Feststoffteile setzen sich auf der Biomasse als Schwimmschicht fest. Dadurch wird der Abfluß des Biogases behindert oder sogar gestoppt. Der sich so verändernde Verarbeitungsprozeß überhöht die Temperatur in dem Säurereaktor und Biogasbehälter und unterbricht dadurch den Vergasungsprozeß. Andererseits sinken andere Teile der Biomasse mit hohem Feststoffgehalt ab. Sie sammeln sich am Boden des Biogasbehälters und bilden dort eine Sinkschicht. Da sich die entgaste und verflüssigte Gülle gleichfalls im unteren Drittel des Biogasbehälters sammelt, besteht die Gefahr einer Verstopfung des Abflußrohres für die entgaste Gülle in den Endlagerbehälter. Das hat zur Folge, daß die Biogasproduktion nachläßt. Sowohl die Schwimm- als auch die Sinkschicht beeinflussen also die Biogasausbeute beträchtlich. Das macht sich insbesondere bei Durchflußverfahren mit täglich neuer Biomassebeschickung bemerkbar, deren Erwärmung im Reaktorkern stattfindet. Im Reaktorkern schließlich sammeln sich gleichzeitig je nach Fütterungsart, insbesondere bei Geflügelgülle, am Boden aus Sand und Kalk bestehende Festteile. Diese müssen nach einigen Monaten entleert und ausgewaschen werden, so daß die Anlage während ihrer Säuberung stillgesetzt werden muß.

Aus der DE-A-2 939 169 und FR-A-2 480 062 ist ein Biogasbehälter bekannt, bei welchem im Innern des Faulbehälters ein mit einer Umwälzpumpe versehenes Rohr angeordnet ist, dessen untere Öffnung sich im unteren Teil des Faulbehälters befindet und dessen obere Öffnung eine Spritzdüse aufweist, die auf die Oberfläche der zu vergasenden Biomasse gerichtet ist. Durch Aufspritzen von Biomasse aus dem Bereich der Sinkschicht auf die Schwimmschicht wird diese zum Absinken gebracht. Die bekannte Anlage arbeitet jedoch unbefriedigend, weil die Sinkschicht zu einem Verstopfen der Absaug- und Ableitungsrohre führen kann und weil in regelmäßigen Abständen unangenehme Wartungs- und Säuberungsarbeiten erforderlich sind, um die Anlage im betriebsbereiten Zustand zu halten.

Zwar ist in FR-A-2 480 062 von einem Verwirbeln (« brassage ») die Rede, um eine Ablagerung von Feststoffen auf dem Boden des Behälters zu verhindern bzw. zu beseitigen. Diese wird aber nur im Sinne von DE-A-2 939 169 durch Absaugen durchgeführt und möglicherweise durch die konische Bodenausbildung verbessert.

Aufgabe der Erfindung ist es daher, durch Beseitigung der Schwimm- und Sinkschichten eine intensivere Verarbeitung der Biomasse bei kontinuierlichem und nahezu wartungsfreiem Betrieb der Verarbeitungsanlagen zu erreichen.

Erfindungsgemäß wird diese Aufgabe bei einem Verfahren der im Patentanspruch 1 angegebenen Art. Durch das erfindungsgemäße Verfahren wird eine Verstopfung des zu dem Endlagerbehälter führenden Abflußrohres für die entgaste Gülle vermieden, dessen Einflußöffnung sich in dem unteren Bereich des Faulbehälters befindet. Durch das erfindungsgemäße Verfahren wird die Bildung dickerer Sinkschichten vermieden, die die Einflußöffnung verstopfen könnten. Nach dem erfindungsgemäßen Verfahren wird die Schwimmschicht durch eine Sinkschichtpartikel enthaltende Flüssigkeit beschwert, so daß die Schwimmschicht in tiefere Teile des Faulraums gedrückt und dort von anaeroben Bakterien weiter zu Biogas verarbeitet wird. Da die gesamte Biogasmasse einschließlich der Sinkschichten ständig im Kreislauf geführt wird, wird bei Erhöhung der Biogasproduktion eine Verstopfung des Abflußrohres vermieden.

Durch die erfindungsgemäße Art der Verwirbelung bildet sich eine mit Sinkschichtpartikeln angereicherte Flüssigkeit, die abgesaugt und auf die Schwimmschicht aufgespritzt werden kann. Durch diese Art der Verwirbelung wird die Biomassesinkschicht gleichzeitig verdünnt, so daß sie sich besser umwälzen läßt.

Zweckmäßigerweise wird die Biomassesinkschicht erwärmt, so daß durch den thermischen Auftrieb eine Sinkschichauflockerung unterstützt wird.

In weiterer Ausgestaltung der Erfindung ist vorgesehen, daß in bestimmten Abständen eine sich in dem Steigrohr ablagernde Sinkschicht, die aus verschiedenen Futterarten zugemischten mineralischen Stoffen, wie Kalk, Sand, Mergel oder dergleichen, besteht, aufgewirbelt und zur Entfernung der Feststoffe abgesaugt wird. Durch diese Art der Entfernung der sich in dem Steigrohr ablagernden Sinkschicht wird die in regelmäßigen Abständen erforderliche Entleerung und Säuberung der Anlage überflüssig.

Nach einer erfinderischen Weiterentwicklung ist vorgesehen, daß die aufgewirbelte Biomassesinkschicht in etwa tangentialer und zur Horizontalen leicht geneigter Richtung auf die Schwimmschicht aufgespritzt wird. Dadurch wird die Schwimmschicht in eine kreisende Bewegung versetzt und sinkt schraubenlinienförmig ab.

Eine Vorrichtung zur Durchführung des erfindungsgemäßen Verfahrens, bestehend aus einem Faulbehälter mit Zu- und Ableitungen für die Biomasse sowie mit einer Ableitung für das entstandene Biogas und aus einem in dem Faulbehälter etwa zentral angeordneten Steigrohr, in dessen unteren Bereich die Zuleitung für die Biomasse mündet, mit im Faulbehälter und im Steigrohr angeordneten Heizspiralen, die durch Thermostaten steuerbar sind, und mit einem im unteren Bereich des Faulbehälters angeordneten Saugrohr, das an eine Pumpe angeschlossen ist, deren Druckseite mit einem im oberen Bereich des Faulbehälters mündenden Rohr versehen ist, das eine auf die Schwimmschicht gerichtete Spritzdüse aufweist, zeichnet sich erfindungsgemäß dadurch aus, daß der das Steigrohr umgebende Boden des Faulbehälters durch eine schräg verlaufende Platte gebildet ist und daß im tiefsten Bereich der Platte zwei Rohre münden, die durch eine Pumpe miteinander verbunden sind, so daß durch ein Rohr Biomasse angesaugt und durch das andere mit einer Strahldüse versehene Rohr wieder mit zur Verwirbelung der Sinkschicht ausreichender Geschwindigkeit in den Faulbehälter eingeleitet wird. Die schrägstehende Bodenplatte bildet eine Prallplatte für den auftreffenden Strahl, so daß eine gute Verwirbelung der Sinkschicht erreicht wird. Das mit der Spritzdüse versehene Rohr kann ebenfalls tangential gegen die Bodenplatte angestellt sein.

Zweckmäßigerweise ist das Saugrohr oberhalb der Strahldüse angeordnet und das mit der Strahldüse versehene Rohr ist etwa horizontal auf die schrägstehende Bodenplatte gerichtet.

Die Druckseite der Pumpe kann durch ein Ventil unter Sperrung der mit der Strahldüse versehenen Leitung auf die die Biomasse auf die Schwimmschicht spritzende Leitung umschaltbar sein. Die Umschaltung kann auch umgekehrt in der Weise erfolgen, daß die aufgewirbelte und verdünnte Sinkschicht durch die mit der Strahldüse versehene Leitung angesaugt wird.

Weitere vorteilhafte Ausgestaltungen der Erfindung sind in den Unteransprüchen beschrieben worden.

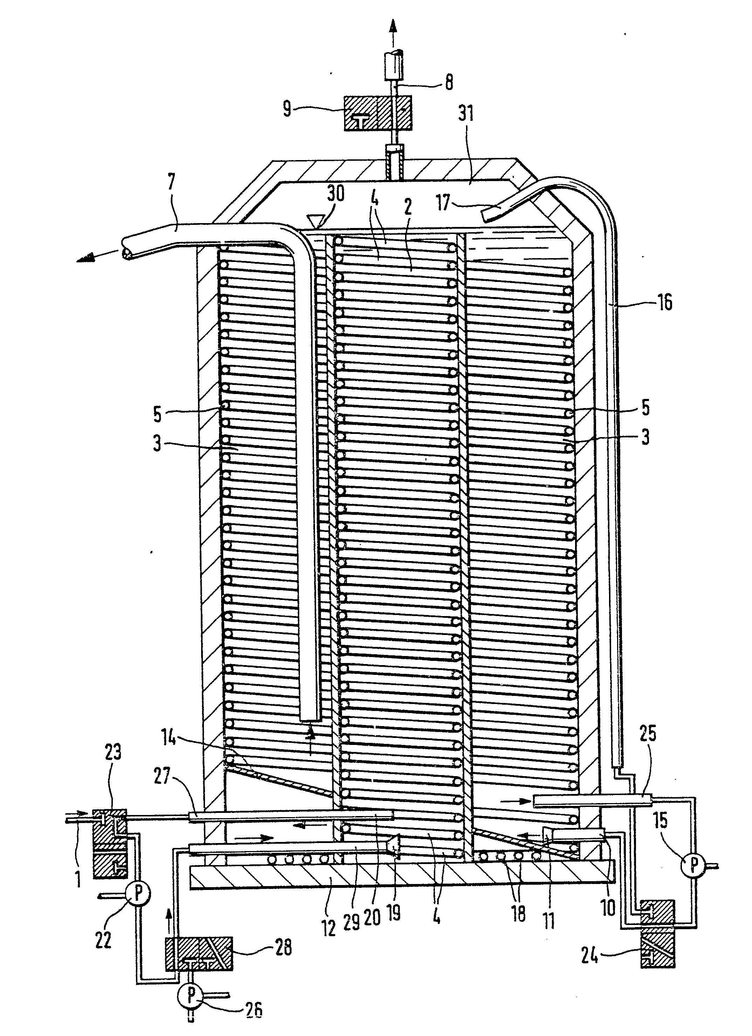

Ein Ausführungsbeispiel der Erfindung wird nachstehend anhand der Zeichnung, in deren einziger Figur ein Längsschnitt durch einen Faulbehälter schematisch dargestellt ist, näher erläutert.

Gülle mit einem Feststoffgehalt bis zu 12 % wird aus einem nicht dargestellten Vorlagerbehälter von unten durch ein Rohr 1 und das Wegeventil 23 in das Steigrohr 2 gepumpt, das im Innern des Faulbehälters 3 angeordnet ist. Das Steigrohr 2 ist so dimensioniert, daß die täglich eingepumpte Güllemenge sich etwa drei Tage in ihm befindet und anschließend über den oberen Rand des Steigrohrs in den Ringraum des umgebenden Faulbehälters 3 strömt. Während dieser Zeit wird die Gülle mittels einer im Steigrohr 2 angeordneten Heizschlange 4 auf die erforderliche Reaktionstemperatur gebracht. Im Faulbehälter 3 sorgt eine an der Innenwandung angeordnete Heizspirale 5 für den Ersatz der entstehenden Wärmeabstrahlungsverluste, so daß die Temperatur im Faulbehälter immer auf der gewünschten Temperatur gehalten werden kann.

In dem Steigrohr 2 wird die Gülle in den Zustand versetzt, in dem die Biogasproduktion voll anlaufen kann, wobei gleichzeitig eine teilweise Zersetzung der organischen Stoffe erfolgt. Der pH-Wert der Gülle, der von Haus aus zwischen 7,5 und 8,5 liegt, wird innerhalb des ersten Tages im Steigrohr durch die Erwärmung und die erste Verarbeitung bis auf einen pH-Wert 5 gesenkt. Durch die im Faulbehälter 3 ablaufende Reaktion wird der pH-Wert nachfolgend auf 6,9 bis 7,1 angehoben.

Im Faulbehälter 3 wird bei Temperaturen zwischen 30° bis 56 °C unter Ausschluß von Sauerstoff die Biomasse in Biogas (2/3 $CH_4$, 1/3 $CO_2$) verwandelt, wobei die Feststoffe weitgehend verarbeitet werden. Hierbei ist es zur gleichmäßigen Gaserzeugung wichtig, daß die Temperatur im Innern des Faulbehälters 3 konstantgehalten wird. Dies erfolgt über die Heizspirale 5 und eine Umwälzpumpe, die mittels eines Thermostaten geregelt werden.

Bei der Gasproduktion ändert sich das spezifische Gewicht der Biomasse je nach fortschreitendem Verarbeitungsverlauf. Die leichten Feststoffe sammeln sich an der Oberfläche und setzen sich dort als Schwimmschicht fest. Dies hat zur Folge, daß eine unerwünschte Temperaturerhöhung eintritt, wodurch der Vergasungsprozeß unterbrochen wird. Andere Teile der Biomasse mit hohem Feststoffgehalt sammeln sich am Boden des Faulbehälters und bilden dort eine Sinkschicht. Da sich die entgaste und verflüssigte Gülle gleichfalls im unteren Drittel des Biobehälters sammelt, besteht die Gefahr einer Verstopfung des Abflußrohres 7 für die entgaste Gülle in den nicht dargestellten Endlagerbehälter. Die Einflußöffnung des Abflußrohres 7 befindet sich im Bereich des unteren Drittels des Biogasbehälters. Eine Verstopfung der Einflußöffnung hätte zur Folge, daß die Biogasproduktion nachläßt.

Im Säurereaktor 2 sammeln sich gleichzeitig je nach Fütterungsart, insbesondere bei Geflügelgülle, zugemischte mineralische Stoffe, wie Kalk, Sand, Mergel und dergleichen, am Boden und bilden dort gleichfalls eine Sinkschicht.

Der aus dem Faulbehälter 3 und dem Säurereaktor 2 bestehende Biogasbehälter gibt das gebildete Biogas an einen in der Zeichnung nicht
dargestellten Gasverbraucher ab. Das Gas wird
über die Leitung 8 abgezogen, die mit einem
Ventil 9 versehen ist.

Die entgaste Gülle sammelt sich aufgrund ihres
spezifischen Gewichts im unteren Drittel des
Faulbehälters 3 und wird dort durch den Überdruck, der durch das Einpumpen der frischen
Gülle in den Säurereaktor 2 in dem Faulbehälter 3
entsteht, über das Ableitungsrohr 7 einem nicht
dargestellten Lagerbehälter zugeführt und findet
von dort Verwendung als hochwertiger Dünger.

Der Säurereaktor 2 ist oben offen, so daß die
täglich eingepumpte Güllemenge nach drei Tagen durch Überlauf in den Ringraum des umgebenden Faulbehälters 3 eintreten kann.

Im Faulraum 3 ist eine das Steigrohr 2 umgebende, schräg zu der Basisplatte 12 angeordnete
Bodenplatte 14 vorgesehen, die den Boden des
Faulbehälters bildet. Diese schräg gestellte Bodenplatte 14 bildet eine Prallplatte und besteht
aus Stahl, auf der sich die Sinkschicht mit hohem
Biomassetrockenanteil absetzt. Diese Sinkschicht wird mit Hilfe der Hochdruckdüse 11
aufgewirbelt und verdünnt und anschließend mittels der Umwälzpumpe 15 über eine Steigleitung
16 durch die Spritzdüse 17 auf die Schwimmschicht aufgespritzt, so daß die durch die Sinkschichtpartikel beschwerte Schwimmschicht beschwert und in den unteren Teil des Faulraums
absinkt, wo sie von den anaeroben Bakterien
weiter zu Biogas verarbeitet wird.

Die Spritzdüse 11 ist an dem vorderen Ende
eines Rohres 10 angeordnet, das über das Wegeventil 24 mit der Druckseite der Pumpe 15 verbunden ist, deren Saugseite mit dem Rohr 25
verbunden ist, das Biomasse aus dem Faulbehälter 3 oberhalb der Spritzdüse 11 absaugt.
Durch das Wegeventil 24 kann wahlweise die
durch das Rohr 25 angesaugte Biomasse zur
Aufwirbelung und Verdünnung der Sinkschicht
durch die Strahldüse 11 wieder in den Faulbehälter eingepumpt oder aber über das Steigrohr
16 und die Spritzdüse 17 auf die Schwimmschicht
aufgespritzt werden. Die Strahldüse 11 ist derart
auf die schrägstehende Bodenplatte 14 gerichtet,
daß die Sinkschicht von dieser abgelöst und
aufgewirbelt wird.

Normalerweise wird die Gülle durch die Leitung
1, das Wegeventil 23 und die Leitung 27 in den
unteren Bereich des Steigrohrs 2 eingeleitet. Zur
Aufwirbelung der sich in dem Steigrohr 2
bildenden Sinkschicht kann in der Weise durch
das Wegeventil 23 und die Pumpe 22 eine Umwälzung stattfinden, daß die durch das Rohr 27
angesaugte Biomasse über die Pumpe 22 und das
Wegeventil 28 durch das Rohr 29 wieder in den
unteren Bereich des Steigrohrs 2 eingeleitet wird.
Das Rohr 29 ist mit einer Strahldüse 19 versehen.

Nachdem die in dem Steigrohr 2 gebildete
Sinkschicht aufgewirbelt worden ist, wird das
Wegeventil 28 in der Weise umgeschaltet, daß die
mit mineralischen Feststoffen angereicherte
Sinkschicht durch die Pumpe 26 abgezogen wird.

Unter der Bodenplatte 14 ist eine Heizspirale 18
angeordnet. Diese wird durch Wasser auf eine
Temperatur von etwa 40 °C erwärmt, so daß die
sich auf der Bodenplatte 14 bildende Biomassesinkschicht durch thermischen Auftrieb gelockert
wird.

Der obere Rand des zentralen Steigrohrs 2 liegt
dicht unterhalb des Güllespiegels 30. Der nach
außen gerichtete Zweig des Steigrohrs 7 liegt
etwa in Höhe des Güllespiegels 30.

Der Gasraum 31 oberhalb des Güllespiegels
beträgt etwa ein Sechstel des Behältervolumens.

Gegenüber der eingeleiteten Gülle weist die
entgaste Gülle etwa einen um 50 % verringerten
Feststoffanteil auf.

**Patentansprüche**

1. Verfahren zur Gcwinnung von Biogas aus
einer Biomasse, wie Gülle, einem Kot, Schlachtereiabfälle und Flüssigkeit enthaltendem Gemisch,
durch anaeroben Abbau in einem alkalischen
Medium in einem geschlossenen Raum, bei dem
die sich unten ablagernde schwere Biomassesinkschicht abgesaugt und auf die sich oben
bildende Biomasseschwimmschicht aufgebracht
wird, dadurch gekennzeichnet, daß die Biomassesinkschicht vor ihrem Absaugen durch den
Strahl einer Flüssigkeit aufgewirbelt wird, die
oberhalb der Sinkschicht aus dem Raum abgesaugt worden ist.

2. Verfahren nach Anspruch 1, dadurch gekennzeichnet, daß die Biomassesinkschicht erwärmt wird.

3. Verfahren nach einem der Ansprüche 1 oder
2, dadurch gekennzeichnet, daß die Biomasse vor
der Behandlung im neutralen bis alkalischen
Medium einer Säuerung unterworfen wird.

4. Verfahren nach einem der Ansprüche 1 bis 3,
dadurch gekennzeichnet, daß in vorherbestimmten Abständen eine sich in dem Steigrohr ablagernde Sinkschicht aus verschiedenen Futterarten zugemischten mineralischen Stoffen, wie
Kalk, Sand, Mergel oder dergleichen, aufgewirbelt und zur Entfernung der Feststoffe abgesaugt wird.

5. Verfahren nach einem der Ansprüche 1 bis 4,
dadurch gekennzeichnet, daß die aufgewirbelte
Biomassesinkschicht in etwa tangentialer und zur
Horizontalen leicht geneigter Richtung auf die
Schwimmschicht aufgespritzt wird.

6. Vorrichtung zur Durchführung eines Verfahrens nach einem der Ansprüche 1 bis 5,
bestehend aus einem Faulbehälter mit Zu- und
Ableitungen für die Biomasse sowie mit einer
Ableitung für das entstandene Biogas und aus
einem in dem Faulbehälter etwa zentral angeordneten Steigrohr, in dessen unterem Bereich die
Zuleitung für die Biomasse mündet, mit im
Faulbehälter und im Steigrohr angeordneten
Heizspiralen, die durch Thermostaten steuerbar
sind, und mit einem im unteren Bereich des
Faulbehälters angeordneten Saugrohr, das an
eine Pumpe angeschlossen ist, deren Druckseite mit einem im oberen Bereich des Faulbehälters mündenden Rohr versehen ist, das eine auf die Schwimmschicht gerichtete Spritzdüse aufweist, dadurch gekennzeichnet, daß der das Steigrohr (4) umgebende Boden des Faulbehälters (3) durch eine schräg verlaufende Platte (14) gebildet ist und daß im tiefsten Bereich der Platte (14) zwei Rohre (10, 25) münden, die durch eine Pumpe (15) miteinander verbunden sind, so daß durch ein Rohr Biomasse angesaugt und durch das andere mit einer Strahldüse (11) versehene Rohr wieder mit einer zur Verwirbelung der Sinkschicht ausreichenden Geschwindigkeit in den Faulbehälter (3) eingeleitet wird.

7. Vorrichtung nach Anspruch 6, dadurch gekennzeichnet, daß das Saugrohr (25) oberhalb der Strahldüse (11) angeordnet ist und daß das mit der Strahldüse (11) versehene Rohr (10) etwa horizontal auf die schräg stehende Bodenplatte (14) gerichtet ist.

8. Vorrichtung nach Anspruch 6 oder 7, dadurch gekennzeichnet, daß die Druckseite der Pumpe (15) durch ein Ventil (24) unter Sperrung der mit der Strahldüse (11) versehenen Leitung (10) auf die die Biomasse auf die Schwimmschicht spritzende Leitung (16) umschaltbar ist.

9. Vorrichtung nach einem der Ansprüche 6 bis 8, dadurch gekennzeichnet, daß das mit der Spritzdüse (17) versehene Rohr (16) etwa schraubenlinienförmig tangential mit leichter Neigung zur Schwimmschicht angeordnet ist.

10. Vorrichtung nach einem der Ansprüche 6 bis 9, dadurch gekennzeichnet, daß unterhalb der schräg angeordneten Bodenplatte (14) Heizeinrichtungen (18) angeordnet sind.

11. Vorrichtung nach einem der Ansprüche 6 bis 10, dadurch gekennzeichnet, daß in den unteren Bereich des Steigrohrs (2) ein mit einer Spritzdüse (19) versehenes Rohr (29) mündet, das mit der Pumpe (22) verbunden ist, deren Saugseite über ein Wegeventil (23) unter Sperrung der Zufuhr von Biomasse mit dem in das Steigrohr (2) mündenden Zuleitungsrohr (27) verbindbar ist.

12. Vorrichtung nach einem der Ansprüche 6 bis 11, dadurch gekennzeichnet, daß eines der in das Steigrohr (2) mündenden Rohre (27, 29) über ein Wegeventil (28) mit einer Saugpumpe (26) verbindbar ist.

**Claims**

1. A process of producing biogas from a biomass, such as liquid manure, excrements, butchery waste, and a liquid-containing mixture by an anaerobic decomposition in an alkaline medium in an enclosed space, wherein the layer of settled heavy biomass deposited at the bottom is sucked off and applied to the layer of floating biomass forming at the top, characterized in that the layer of settled biomass is agitated by a liquid jet before the layer of settled biomass is sucked off and said jet consists of liquid that has been sucked from the space above the settled layer.

2. A process according to claim 1, characterized in that the mass of settled biomass is heated.

3. A process according to claim 1 or 2, characterized in that the biomass is acidified before it is treated in the neutral to alkaline medium.

4. A process according to any of claims 1 to 3, characterized in that a layer settling in the rising pipe and consisting of mineral substances which are admixed to certain kinds of animal feed, such as lime, sand, marl or the like, is agitated at certain intervals of time and is sucked off to remove the solids.

5. A process according to any of claims 1 to 4, characterized in that the agitated layer of settled biomass is sprayed onto the floating layer in an approximately tangential direction, which is slightly inclined to the horizontal.

6. Apparatus for carrying out a process according to any of claims 1 to 5, comprising a digesting tank provided with lines for supplying and withdrawing the biomass and with a line for withdrawing the biogas which has been formed, also comprising a rising pipe, which is approximately centrally disposed in the digesting tank, wherein the line for supplying the biomass opens into the lower portion of the rising pipe, also comprising heating coils, which are disposed in the digesting tank and in the rising pipe and are controllable by thermostats, and comprising a suction pipe, which is disposed in the lower portion of the digesting tank and connected to a pump, the pressure side of which is provided with a pipe that opens into the upper portion of the digesting tank and comprises a spray nozzle that is directed toward the floating layer, characterized in that the bottom of the digesting tank (3) surrounds the rising plate (4) and consists of an inclined plate (14) and that two pipes (10, 25) are provided, which open into the lowermost portion of the plate (14) and are interconnected by a pump (15) so that biomass is sucked by one pipe and through the other pipe, which is provided with a jet nozzle (11), is re-introduced into the digesting tank (3) at a velocity which is sufficient to agitate the settled layer.

7. Apparatus according to claim 6, characterized in that the suction pipe (25) is disposed above the jet nozzle (11) and the pipe (10) provided with the jet nozzle (11) is approximately horizontally directed towards the inclined bottom plate (14).

8. Apparatus according to claim 6 or 7, characterized in that the pressure side of the pump (15) is adapted to be connected by a valve (24) to the line (16) for spraying the biomass onto the floating layer and said valve blocks the line (10) provided with the jet nozzle (11) at the same time.

9. Apparatus according to any of claims 6 to 8, characterized in that the pipe (16) provided with the jet nozzle (17) extends tangentially approximately along a helix and with a slight inclination towards the floating layer.

10. Apparatus according to any of claims 6 to 9, characterized in that heating means (18) are provided below the inclined bottom plate (14).

11. Apparatus according to any of claims 6 to 10, characterized in that a pipe (29), which is provided with a spray nozzle (19) opens into the lower portion of the rising pipe (2) and is connected to the pump (22), the suction side of which is adapted to be connected by a directional valve (23) to the supply pipe (27) opening into the rising pipe (2), and said directional valve blocks the supply of biomass at the same time.

12. Apparatus according to any of claims 6 to 11, characterized in that one of the pipes (27, 29) which open into the rising pipe (2) is adapted to be connected by a directional valve (28) to a vacuum pump (26).

**Revendications**

1. Procédé pour la production de biogaz à partir d'une biomasse telle que du lisier, un mélange contenant des excréments, des déchets d'abattoir et du liquide, consistant dans la décomposition anaérobie dans un milieu alcalin se trouvant dans un espace fermé, dans lequel la biomasse lourde décantée se déposant sur le fond est aspirée et appliquée sur la couche flottante de biomasse se formant sur le dessus, caractérisé en ce que la couche de biomasse décantée est soulevée en tourbillons par le jet d'un liquide avant d'être aspirée, le liquide ayant été aspiré d'au-dessus de la couche de décantation hors de l'espace fermé.

2. Procédé selon la revendication 1 caractérisé en ce que la couche de biomasse décantée est réchauffée.

3. Procédé selon la revendication 1 ou 2 caractérisé en ce que la biomasse est soumise à une acidification avant d'être traitée dans le milieu neutre à alcalin.

4. Procédé selon l'une des revendications 1 à 3 caractérisé en ce qu'à intervalles prédéterminés, une couche de décantation se composant de substances minérales, telles que de la chaux, du sable, de la marne ou des substances semblables qui ont été ajoutées à divers types de fourrages et se déposant dans la conduite montante est soulevée en tourbillons et est aspirée pour éliminer les solides.

5. Procédé selon l'une des revendications 1 à 4 caractérisé en ce que la couche de biomasse décantée soulevée en tourbillons est giclée sur la couche flottante dans une direction approximativement tangentielle et légèrement inclinée par rapport à l'horizontale.

6. Dispositif pour l'exécution d'un procédé selon les revendications 1 à 5 consistant en une cuve de putréfaction comportant des conduites d'amenée et d'évacuation de la biomasse ainsi qu'une conduite d'évacuation du biogaz qui s'est formé et une conduite montante disposée approximativement au centre de la cuve de putréfaction, dans laquelle débouche dans la partie inférieure la conduite d'amenée de la biomasse, le dispositif comportant, par ailleurs, des spirales de chauffages qui sont disposées dans la cuve de putréfaction et dans la conduite montante et peuvent être commandées par thermostat, et un tuyau d'aspiration qui est disposé dans la partie inférieure de la cuve de putréfaction et est raccordé à une pompe dont le côté pression est pourvu d'un tuyau qui débouche dans la partie supérieure de la cuve de putréfaction et est pourvu d'un gicleur orienté vers la couche flottante, caractérisé en ce que le fond de la cuve de putréfaction (3) qui entoure la conduite montante (4) est formé par une plaque (14) orientée de manière oblique et que dans la partie la plus basse de la plaque (14) débouchent deux tuyaux (10, 25) qui sont raccordés entre eux par une pompe (15) de manière à ce que la biomasse est aspirée à travers un tuyau et est retournée dans la cuve de putréfaction (3) à une vitesse suffisante pour soulever en tourbillons la couche de décantation à travers l'autre tuyau qui est pourvu d'une tuyère (11).

7. Dispositif selon la revendication 6 caractérisé en ce que le tuyau d'aspiration (25) est disposé au-dessus de la tuyère (11) et que le tuyau (10) pourvu de la tuyère (11) est orienté à peu près horizontalement vers la plaque de fond (14) placée obliquement.

8. Dispositif selon la revendication 6 ou 7 caractérisé en ce que le côté pression de la pompe (15) peut être commuté au moyen d'une valve (24) bloquant la conduite (10) pourvue de la tuyère (11) sur la conduite (16) giclant la biomasse sur la couche flottante.

9. Dispositif selon l'une des revendications 6 à 8 caractérisé en ce que le tuyau (16) pourvu du gicleur (17) est disposé tangentiellement approximativement le long d'une hélice et avec une légère inclinaison vers la couche flottante.

10. Dispositif selon l'une des revendications 6 à 9 caractérisé en ce qu'au-dessous de la plaque de fond (14) disposée de manière oblique sont prévus des moyens de chauffage (18).

11. Dispositif selon l'une des revendications 6 à 10 caractérisé en ce que dans la partie inférieure du tuyau montant (2) débouche un tuyau (29) pourvu d'un gicleur (19), qui est raccordé à la pompe (22) dont le côté aspiration peut être raccordé (22) dont le côté aspiration peut être raccordé au moyen d'un distributeur (23) bloquant l'amenée de la biomasse avec le tuyau d'amenée (27) débouchant dans la conduite montante (2).

12. Dispositif selon l'une des revendications 6 à 11 caractérisé en ce qu'un des tuyaux (27, 29) débouchant dans la conduite montante (2) peut être raccordé au moyen d'un distributeur (28) à une pompe d'aspiration (26).